# EUROPEAN PATENT SPECIFICATION

(11) **EP 1 931 322 B1**
(45) Date of publication and mention of the grant of the patent: **15.08.2012**
(21) Application number: 06813899.9
(22) Date of filing: 28.08.2006
(51) Int. Cl.: A61K 31/00, A61K 31/505, A61K 31/506, A61K 45/06, A61P 15/00

(54) **IKK INHIBITORS FOR THE TREATMENT OF ENDOMETRIOSIS**
IKK-HEMMER ZUR BEHANDLUNG VON ENDOMETRIOSE
INHIBITEURS D'IKK POUR LE TRAITEMENT DE L'ENDOMETRIOSE

(30) Priority: 07.09.2005 US 714645 P; 11.10.2005 EP 05109450
(43) Date of publication of application: 18.06.2008
(60) Divisional application: 10184024.7
(73) Proprietor: Merck Serono SA, 1267 Coinsins, Vaud (CH)
(72) Inventor: PALMER, Stephen, S., Plympton, Massachusetts 02367 (US); NATARAJA, Selvaraj, Randolph, Massachusetts 02368 (US)
(74) Representative: Merck Serono SA - Geneva Intellectual Property
(86) International application number: PCT/US2006/033686
(87) International publication number: WO 2007/030362

(56) References cited:
- EP-A1- 1 424 080
- WO-A-2004/048343
- WO-A2-02/46171
- WO-A2-2004/007457
- WO-A2-2005/034978
- BUHLER K: "ENDOMETRIOSE, MEDIKAMENTOESE THERAPIE MEDICAL TREATMENT OF ENDOMETRIOSIS" TW-GYNAEKOLOGIE, KARLSRUHE, DE, vol. 10, no. 6, 1997, pages 271-274, XP008074036 ISSN: 0935-3208
- KARCK U: "ENDOMETRIOSE ENDOMETRIOSIS" GYNAEKOLOGE, SPRINGER VERLAG, BERLIN, DE, vol. 30, no. 1, 1997, pages 581-594, XP008074038 ISSN: 0017-5994
- YIN M J ET AL: "The anti-inflammatory agents aspirin and salicylate inhibit the activity of I(kappa)B kinase-beta." NATURE 5 NOV 1998, vol. 396, no. 6706, 5 November 1998 (1998-11-05), pages 77-80, XP002416699 ISSN: 0028-0836

## Description

### Background of the Invention

Endometriosis is one of the most frequent diseases of women in their reproductive lifespan. It is characterized by the presence of endometrial tissue outside the uterine cavity, consisting histological of glands and stroma. The anatomical sites most often affected are the ovaries, uterosacral ligaments, pelvic peritoneum, rectovaginal septum, cervix, vagina, the fallopian tubes and vulva.

Endometriosis is considered to be a benign disease, but endometriotic lesions occasionally become malignant. As in other kind of malignancies, the development of endometriosis-derived neoplasms is due to concurrent events, involving alterations in growth factors and/or oncogenes regulation (Kyama e al. 2003). Further, endometriosis is considered as a major cause of infertility (Giudice et al. 2004).

The current treatment of endometriosis consists of hormonal therapy and/or surgery. Hormonal therapies include high dose of progestogens, oral contraceptives (combinations of estrogen and progesterone), Danazol (an androgenic derivative of ethisterone) and more recently GnRH agonists. These hormonal therapies are effective on pelvic pain and may induce an objective regression of lesions, but have several caveats. Estrogen may stimulate and cause proliferation of endometriotic tissue since it may be unable to respond to progesterone (Dawood et al, 1993). Progestational agents can provoke irregular bleeding along with depression, weight gain, and fluid retention. Danazol can improve symptoms in approximately 66-100% of the patients suffering from pain, but recurrence rates after up to 4 years are approximately 40% - 50%. Other drawbacks of Danazol therapy are weight gain and androgenic side effects. GnRH analogs are more potent and long acting than native GnRH, which act by removing the estrogenic stimulus for the growth of all estrogen sensitive tissues. Side effects of GnRH analogs are mainly secondary to the profound hypoestrogenemia, like decreased bone density, and recurrence rate are up to 50% after 5 years (Waller et al., 1993).

Surgical intervention can be conservative, if fertility is desired, or can lead to the removal of the uterus, tubes and ovaries in case of severe disease. In any case, even limited surgical treatment leads to a significant decrease in fertility.

Although endometriosis stands as one of the most investigated disorders of gynecology, the current understanding of pathophysiology of the disease remains elusive. According to a favored theory, endometriotic lesions develop by eutopic endometrical cells leaving their primary site, possibly by retrograde menstruation, and implant at distant sites, followed by invasion of host tissue and proliferation. Furthermore, it appears that endometriosis is an invasive and metastasizing disease. Though endometriotic cells proliferate to a certain extent, they are not neoplastic as typically found in carcinomas. Apparently, endometriotic cells become senescent, apoptotic and necrotic. Inflammatory responses that are induced or accompanied by lesion formation finally lead to fibrosis and the formation of scars.

Recently, it has been demonstrated that recombinant human TNFα binding protein (rh-TBP-1) is effective in reducing the size and severity of endometriotic lesions in an experimental model of endometriosis (D'Hooghe et al. 2001). These results were the first to demonstrate that an anti-inflammatory molecule (r-hTBP-1) that targeted the TNF-α pathway provided effective medical treatment for patients with endometriosis that did not inhibit ovulation.

IκB kinases (IKKs) are key regulatory signaling molecules coordinating the activation of NF-kB. The activity of IκB kinase (IKK) was found to reside in a high molecular weight (>600 kD) complex that contained two kinase subunits (IKK1 or α and IKK2 or β). Both share significant sequence homology and contain identical structural domains. Through their leucine-zipper domains, IKK1 and IKK2 interact to form hetero- and homodimers in vitro, although only heterodimers are found in vivo. A third protein in the IKK complex, IKK3 or y (also called NEMO or IKKAP), which does not contain a catalytic kinase domain, preferentially associates with IKK2 and is required for the activation of IKK1-IKK2 heterodimers in response to the proinflammatory cytokines tumor necrosis factor-a (TNF)-a and interleukin-1 (IL-1) (Mercurio F et al. (1997).

The invention described herein clearly shows the unexpected result that inhibiting IKK, by means of an IKK inhibitor, reduces endometriosis. The reduction of endometriotic lesions using IKK inhibitors can also improve fertility rates, since the normalization of genital structure has a positive effect on the implantation rate.

### Summary of the invention

The present invention relates to a method of treating and/or preventing endometriosis in an individual comprising administering a therapeutically effective amount of an IKK inhibitor.

The invention further relates to a method of treating and/or preventing endometriosis by combined treatment of hormonal suppressor (e.g. GnRH antagonists, GnRH agonists, aromatase inhibitors, progesterone receptor modulators, estrogen receptor modulators) along with an IKK inhibitor.

The invention also relates to a method of treating endometriosis-related infertility in a female comprising the administration of a therapeutically effective amount of an IKK inhibitor, alone or in combination with other fertility drugs.

The invention finally relates to a pharmaceutical composition comprising an IKK inhibitor, a hormonal suppressor and a pharmaceutically acceptable excipient.

### Description of the invention

The following paragraphs provide definitions of the various chemical moieties that make up the compounds according to the invention and are intended to apply uniformly throughout the specification and claims unless an otherwise expressly set out definition provides a broader definition.

"Aryl" refers to an unsaturated aromatic carbocyclic group of from 6 to 14 carbon atoms having a single ring (e.g. phenyl) or multiple condensed rings (e.g. naphthyl). Preferred aryls include phenyl, naphthyl, phenantrenyl.

"Alkylaryl refers to an alkyl having at least one alkyl hydrogen atom replaced with an aryl moiety, such as benzyl, -(CH₂)₂phenyl, -(CH₂)₃phenyl, -CH(phenyl)₂.

"Alkyl" refers to a straight chain or branched, saturated or unsaturated alkyl, cyclic or non-cyclic hydrocarbon having from 1 to 10 carbon atoms, while "lower alkyl" or "C₁-C₆-alkyl"has the same meaning but only has from to 6 carbon atoms. Representative saturated straight chain alkyls include methyl, ethyl, n-propyl, n-butyl, n-pentyl, n-hexyl; while saturated branched alkyls include isopropyl, secbutyl, isobutyl, tert-butyl, isopentyl. Unsaturated alkyls contain at least one double or triple bond between adjacent carbon atoms (also referred to as an "alkenyl" or "alkynyl", respectively). Representative straight chain and branched alkenyls include ethylenyl, propylenyl, 1- butenyl, 2-butenyl, isobutylenyl, 1-pentenyl, 2-pentenyl, 3-methyl-1- butenyl, 2-methyl-2-butenyl, 2,3-dimethyl, 2-butenyl; while representative straight chain and branched alkynyls include acetylenyl, propynyl, 1-butynyl, 2-butynyl, 1-pentynyl, 2-pentynyl, 3- methyl-1-butynyl. Representative saturated cyclic alkyls include cyclopropyl, cyclobutyl, cyclopentyl, cyclohexyl; while unsaturated cyclic alkyls include cyclopentenyl and cyclohexenyl. Cycloalkyls are also referred to herein as "carbocyclic" rings systems, and include bi- and tri-cyclic ring systems having from 8 to 14 carbon atoms, such as a cycloalkyl (such as cyclopentane or cyclohexane) fused to one or more aromatic (such as phenyl) or non-aromatic (such as cyclohexane) carbocyclic rings.

"Alkoxy," refers to -O-(alkyl) or -O-aryl), such as methoxy, ethoxy, n-propyloxy, iso propyloxy, n-butyloxy, iso-butyloxy, phenoxy.

"C₂-C₆-alkenyl" refers to alkenyl groups preferably having from 2 to 6 carbon atoms and having at least 1 or 2 sites of alkenyl unsaturation. Preferable alkenyl groups include ethenyl (-CH=CH₂), n-2-propenyl (allyl, -CH₂CH=CH₂).

"C₂-C₆-alkynyl" refers to alkynyl groups preferably having from 2 to 6 carbon atoms and having at least 1-2 sites of alkynyl unsaturation, preferred alkynyl groups include ethynyl (-C≡CH), propargyl (-CH₂C≡CH).

"Halogen" refers to fluorine, chlorine, bromine or iodine.

"Keto" refers to a carbonyl group (i. e.,C =O).

"Heteroaryl" refers to an aromatic heterocycle ring of 5- to 10 members and 10 having at least one heteroatom selected from nitrogen, oxygen and sulfur, and containing at least 1 carbon atom, including both mono- and bicyclic ring systems. Representative heteroaryls are pyridyl, furyl, benzofuranyl, thiophenyl, benzothiophenyl, quinolinyl, pyrrolyl, indolyl, oxazolyl, benzoxazolyl, imidazolyl, benzimidazolyl, thiazolyl, benzothiazolyl, isoxazolyl, pyrazolyl, isothiazolyl, pyridazinyl, pyrimidinyl, pyrazinyl, triazinyl, cinnolinyl, phthalazinyl, and quinazolinyl.

"Heteroalkylaryl" refers to an alkyl having at least one alkyl hydrogen atom replaced with a heteroaryl moiety, such as -CH₂-pyridinyl, -CH₂-pyrimidinyl .

"Heterocycloalkyl" or "heterocycle"refers to a heterocyclic ring containing from 5 to 10 ring atoms. Specifically, a 5- to 7-membered monocyclic, or 7- to 10-membered bicyclic, heterocyclic ring which is either saturated, unsaturated, or aromatic, and which contains from 1 to 4 heteroatoms independently selected from nitrogen, oxygen and sulfur, and wherein the nitrogen and sulfur heteroatoms may be optionally oxidized, and the nitrogen heteroatom may be optionally quaternized, including bicyclic rings in which any of the above heterocycles are fused to a benzene ring. The heterocycle may be attached via any heteroatom or carbon atom. Heterocycles include heteroaryls as defined above. Thus, in addition to the heteroaryls listed above, heterocycles also include morpholinyl, pyrrolidinonyl, pyrrolidinyl, piperidinyl, piperazinyl, hydantoinyl, valerolactamyl, oxiranyl, oxetanyl, tetrahydrofuranyl, tetrahydropyranyl, tetrahydropyridinyl, tetrahydropyrimidinyl, tetrahydrothiophenyl, tetrahydrothiopyranyl, tetrahydropyrimidinyl, tetrahydrothiophenyl, tetrahydrothiopyranyl.

"Alkylheterocycloalkyl" refers to an alkyl having at least one alkyl hydrogen atom replaced with a heterocycle, such as 2-(1-pyrrolidinyl)ethyl, 4-morpholinylmethyl, (1-methyl-4-piperidinyl)methyl.

The term "substituted" as used herein refers to any of the above groups (i. e. alkyl, aryl, alkyl aryl, heterocyclyl and heterocycloalkyl) wherein at least one hydrogen atom is replaced with a substituent. In the case of a keto substituent ("C(=O)") two hydrogen atoms are replaced. Substituents include halogen, hydroxy, alkyl, substituted alkyl (such as haloalkyl, mono- or all-substituted aminoalkyl, alkyloxyalkyl, aryl, substituted aryl, arylalkyl, substituted arylalkyl, heterocycloalkyl, substituted heterocycloalkyl, alkylheterocycloalkyl, substituted alkylheterocycloalkyl, -NRₐR_{b}, - NRₐC(=O)R_{b},
-NRₐC(=O)NRₐR_{b}, -NRₐC(=O)OR_{b} -NRₐSO₂R_{b}, -ORₐ, -C(=O)Rₐ, -C(=O)ORₐ, -C(=O) -NRₐR_{b}, -OC(=O)Rₐ, -OC(=O)ORₐ, -OC(=O)NRₐR_{b}, -NRₐSO₂R_{b}, or a radical of the formula Y-Z-Rₐ where Y is alkanediyl, substituted alkanediyl, or a direct bond, Z is -O-, -S-,
S(=O)-, -S(=O)₂-, -N(R_{b})-, -C(=O)-, -C(=O)O-, -OC(=O)-, -N(R_{b})C(=O)-, - C(=O)N(R_{b})- or a direct bond, wherein Rₐ and R_{b} are the same or different and independently hydrogen, amino, alkyl, substituted alkyl (including halogenated alkyl), aryl, substituted aryl, alkylaryl, substituted alkylaryl, heterocycloalkyl, substituted heterocycloalkyl, alkylheterocyloalkyl or substituted alkylheterocycloalkyl, or wherein Rₐ and R_{b} taken together with the nitrogen atom to which they are attached form a heterocycle or substituted heterocycle.

"Haloalkyl" refers to an alkyl having one or more hydrogen atoms replaced with halogen, such as -CF₃.

"Hydroxyalkyl" means alkyl having one or more hydrogen atoms replaced with hydroxy, such as -CH₂OH.

"Sulfonyl" refers to group "-SO₂-R" wherein R is selected from H, "aryl", "heteroaryl", "C₁-C₆-alkyl", "C₁-C₆-alkyl" substituted with halogens, e.g., an -SO₂-CF₃ group, "C₂-C₆-alkenyl", "C₂-C₆-alkynyl", "C₃-C₈-cycloalkyl", "heterocycloalkyl", "aryl", "heteroaryl", "C₁-C₆-alkyl aryl" or "C₁-C₆-alkyl heteroaryl", "C₂-C₆-alkenyl aryl", "C₂-C₆-alkenyl heteroaryl", "C₂-C₆-alkynyl aryl", "C₂-C₆-alkynylheteroaryl", "C₁-C₆-alkyl cycloalkyl", "C₁-C₆-alkyl heterocycloalkyl".

"Sulfinyl" refers to a group "-S(O)-R" wherein R is selected from H, "C₁-C₆-alkyl", "C₁-C₆-alkyl" substituted with halogens, e.g., a -SO-CF₃ group, "C₂-C₆-alkenyl", "C₂-C₆-alkynyl", "C₃-C₈-cycloalkyl", "heterocycloalkyl", "aryl", "heteroaryl", "C₁-C₆-alkyl aryl" or "C₁-C₆-alkyl heteroaryl", "C₂-C₆-alkenyl aryl", "C₂-C₆-alkenyl heteroaryl", "C₂-C₆-alkynyl aryl", "C₂-C₆-alkynylheteroaryl", "C₁-C₆-alkyl cycloalkyl", "C₁-C₆-alkyl heterocycloalkyl".

"Sulfanyl" refers to groups -S-R where R includes H, "C₁-C₆-alkyl", "C₁-C₆-alkyl" substituted with halogens, *e.g*., a -SO-CF₃ group; "C₂-C₆-alkenyl", "C₂-C₆-alkynyl", "C₃-C₈-cycloalk", "heterocycloalkyl" "aryl", "heteroaryl", "C₁-C₆-alkyl aryl" or "C₁-C₆-alkyl heteroaryl", "C₂-C₆-alkenyl aryl", "C₂-C₆-alkenyl heteroaryl", "C₂-C₆-alkynyl aryl", "C₂-C₆-alkynylheteroaryl", "C₁-C₆-alkyl .cycloalkyl", "C₁-C₆-alkyl heterocycloalkyl". Preferred sulfanyl groups include methylsulfanyl, ethylsulfanyl, and the like.

"Carboxyl" refers -COOH.

"Amino" refers to the group NRR' where each R,R' is independently hydrogen or "C₁-C₆-alkyl" or "aryl" or "heteroaryl" or "C₁-C₆-alkyl aryl" or "C₁-C₆-alkyl heteroaryl", or "cycloalkyl", or "heterocycloalkyl", and where R and R', together with the nitrogen atom to which they are attached, can optionally form a 3-8-membered heterocycloalkyl ring.

"Ammonium" refers to a positively charged group -N⁺RR'R", where each R,R',R" is independently "C₁-C₆-alkyl" or "C₁-C₆-alkyl aryl" or "C₁-C₆-alkyl heteroaryl", or "cycloalkyl", or "heterocycloalkyl", and where R and R', together with the nitrogen atom to which they are attached, can optionally form a 3-8-membered heterocycloalkyl ring.

"HCl" means the hydrochloride salt of compounds depicted by their chemical structure.

"Nitrogen-containing non-aromatic heterocycle" means morpholinyl, thiomorpholinyl, pyrrolidinonyl, pyrrolidinyl, piperidinyl, homopiperidinyl, piperazinyl, homopiperazinyl, hydantoinyl, tetrahydropyridinyl, tetrahydropyrimidinyl, oxazolidinyl, thiazolidinyl, indolinyl, isoindolinyl, tetrahydroquinolinyl, tetrahydroisoquinolinyl.

"Pharmaceutically acceptable cationic salts or complexes" is intended to define such salts as the alkali metal salts, (e.g. sodium and potassium), alkaline earth metal salts (e.g. calcium or magnesium), aluminium salts, ammonium salts and salts with organic amines such as with methylamine, dimethylamine, trimethylamine, ethylamine, triethylamine, morpholine, N-Me-D-glucamine, N,N'-bis(phenylmethyl)-1,2-ethanediamine, ethanolamine, diethanolamine, ethylenediamine, N-methylmorpholine, piperidine, benzathine (N,N'-dibenzylethylenediamine), choline, ethylene-diamine, meglumine (N-methylglucamine), benethamine (N-benzylphenethylamine), diethylamine, piperazine, thromethamine (2-amino-2-hydroxymethyl-1,3-propanediol), procaine as well as amines of formula -NR,R',R" wherein R, R', R" is independently hydrogen, alkyl or benzyl. Especially preferred salts are sodium and potassium salts.

"Pharmaceutically acceptable salts or complexes" refers to salts or complexes of the below-identified compounds of the present invention that retain the desired biological activity. Examples of such salts include, but are not restricted to acid addition salts formed with inorganic acids (*e.g*., hydrochloric acid, hydrobromic acid, sulfuric acid, phosphoric acid, nitric acid), and salts formed with organic acids such as acetic acid, oxalic acid, tartaric acid, succinic acid, malic acid, fumaric acid, maleic acid, ascorbic acid, benzoic acid, tannic acid, pamoic acid, alginic acid, polyglutamic acid, naphthalene sulfonic acid, naphthalene disulfonic acid, and poly-galacturonic acid. Said compounds can also be administered as pharmaceutically acceptable quaternary salts known by a person skilled in the art, which specifically include the quarternary ammonium salt of the formula NR,R',R" + Z⁻, wherein R, R', R" is independently hydrogen, alkyl, or benzyl, C₁-C₆-alkyl, C₂-C₆-alkenyl, C₂-C₆-alkynyl, C₁-C₆-alkyl aryl, C₁-C₆-alkyl heteroaryl, cycloalkyl, heterocycloalkyl, and Z is a counterion, including chloride, bromide, iodide, -O-alkyl, toluenesulfonate, methylsulfonate, sulfonate, phosphate, or carboxylate (such as benzoate, succinate, acetate, glycolate, maleate, malate, fumarate, citrate, tartrate, ascorbate, cinnamoate, mandeloate, and diphenylacetate).

"Pharmaceutically active derivative" refers to any compound that upon administration to the recipient, is capable of providing directly or indirectly, the activity disclosed herein.

"Aromatase Inhibitors" refers to drugs that inhibit the enzyme aromatase and by that lowers the level of the estradiol. Preferred aromatase inhibitors include by way of example anastrozole, letrozole, vorozole and exemestane.

"Estrogen receptor modulators (SERM)"refers to drugs that block the actions of estrogen by occupying the estrogen receptors on cells. SERMS also include estrogen receptor beta antagonists and estrogen receptor beta agonists. Preferred SERMs include by way of example Tamoxifen, Raloxifen.

"GnRH antagonists" refers to synthetic GNRH, analogues, which are drugs that competitively block the pituitary GnRH receptor, which is located on the plasma membrane of gonadotrophs, inducing a rapid, reversible suppression of gonadotrophin secretion. Preferred GnRH antagonists include by way of example Cetrorelix, Ganirelix.

"GnRH agonists" refers to decapeptide modifications of the natural hormone GnRH, which are drugs that desensitize GnRH receptors of the pituitary gland at continued exposure, which causes an initial stimulation of the pituitary-ovarian axis, followed by a reduction in circulating serum gonadotrophin concentration and inhibition of ovarian function. Preferred GnRH agonists include by way of example Buserelin acetate, Nafarelin, Leuprolide, Triptolerin, Goserelin.

"IKK inhibitor" refers to a compound, a peptide or a protein that inhibits the activity of IκB kinase (IKK). When IKK is inhibited, IKK is unable to exert its enzymatic, biological and/or pharmacological effects. Preferably IKK-2 is inhibited. IKK-2 is a 756 amino acid serine-threonine kinase showing 52 % identity to the structure of IKK-1 ((Mercurio et al. (1997) and (Woronicz et al. (1997)).

"Progesterone receptor modulators (SPRMs)": The progesterone receptor, a member of the superfamily of nuclear receptors, is the receptor for progesterone that plays a pivotal role in female reproduction. Selective progesterone receptor modulators are drugs that can have agonist, antagonist or partial (mixed) agonist/antagonist activities depending upon the site of action. A preferred SPRM includes by way of example asoprisnil.

A first aspect of the present invention is to provide an IKK inhibitor according to claim 1 for use for treating and/or preventing endometriosis in an individual . In a preferred embodiment the individual is a human female.

In a second aspect, the invention relates to an IKK inhibitor according to claim 1 for use for treating and/or preventing endometriosis by sequential or combined treatment of hormonal suppressor (e.g. GnRH antagonists, GnRH agonists, aromatase inhibitors, progesterone receptor modulators, estrogen receptor modulators) along with said an IKK inhibitor.

Second or subsequent administrations of therapeutically effective amounts can be performed at a dosage which is the same, less than or greater than the initial or previous dose administered to the individual. Second or subsequent administrations can be administered during or prior to relapse of the endometriosis or the related symptoms. The terms "relapse" or "reoccurrence" are defined to encompass the appearance of one or more of symptoms of endometriosis.

In a third aspect, the invention relates to an IKK inhibitor according to claim 1 for use for treating endometriosis-related infertility in a female comprising the administration of a therapeutically effective amount of said IKK inhibitor, alone or in combination with other fertility drugs

In one embodiment, the sequential or combined treatment regimen minimizes the disease by suppressing endocrine-dependent cells.

A forth aspect of the present invention consists in a pharmaceutical composition comprising an IKK inhibitor of claim 1 a hormonal suppressor (e.g. GnRH antagonists, GnRH agonists, aromatase inhibitors, progesterone receptor modulators, estrogen receptor modulators) and a pharmaceutically acceptable excipient.

A fifth aspect of the present invention consists in the use of an IKK inhibitor of claim 1 in the manufacture of a medicament for the treatment and/or prevention of endometriosis.

The term "preventing", as used herein, should be understood as partially or totally preventing, inhibiting, alleviating, or reversing one or more symptoms or cause(s) of endometriosis.

A proposed model for progression of endometriotic disease predicts that lesions progress from benign inflammatory lesions responsive to endocrine intervention to partially or completely hormonally unresponsive lesions that involve upregulated survival pathways in addition to inflammatory pathways.

Therefore, in one embodiment, the IKK inhibitor may interfere with survival pathways in endometriosis.

A sixth aspect of the invention relates to the use of an IKK inhibitor of claim 1 together with a hormonal suppressor (e.g. GnRH antagonists, GnRH agonists, aromatase inhibitors, progesterone receptor modulators, estrogen receptor modulators) and a pharmaceutically acceptable carrier in the manufacture of a medicament for the treatment and/or prevention of endometriosis.

The use of the IKK inhibitor together with a hormonal suppressor (e.g. GnRH antagonists, GnRH agonists, aromatase inhibitors, progesterone receptor modulators, estrogen receptor modulators) can be a sequential or a combined use of the IKK inhibitor and the hormonal suppressor.

A seventh aspect of the invention, relates to the use of an IKK inhibitor of claim 1 alone or in combination with other drugs, in the manufacture of a medicament for the treatment of endometriosis-related infertility.

In particular, when endometriosis-related infertility is intended to be treated or cured, drugs for the treatment of infertility e.g. biologically active human chorionic gonadotrophin (hCG), luteinizing hormone (LH) or follicle stimulating hormone (FSH), either in a natural highly purified or in a recombinant form, can be administered. Such molecules and methods of their production have been described in the European Patent Applications EP 160,699, EP 211,894 and EP 322,438.

The pharmaceutical compositions of the present invention can be administered by a variety of routes including oral, rectal, transdermal, subcutaneous, intravenous, intramuscular and intranasal. The compositions for oral administration can take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include prefilled, premeasured ampoules or syringes of the liquid compositions or pills, tablets, capsules in the case of solid compositions. In such compositions, the IKK inhibitor is usually a minor component (from about 0.1 to about 50% by weight or preferably from about 1 to about 40% by weight) with the remainder being various vehicles or carriers and processing aids helpful for forming the desired dosing form.

Liquid forms suitable for oral administration may include a suitable aqueous or nonaqueous vehicle with buffers, suspending and dispensing agents, colorants, flavors .

Solid forms may include, for example, any of the following ingredients, or compounds of a similar nature: a binder such as microcrystalline cellulose, gum tragacanth or gelatine; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavoring agent such as pepper-mint, methyl salicylate, or orange flavoring.

Injectable compositions are typically based upon injectable sterile saline or phosphate-buffered saline or other injectable carriers known in the art. As above-mentioned, the IKK inhibitor in such compositions is typically a minor component, frequently ranging between 0.05 to 10% by weight with the remainder being the injectable carrier.

The above-described components for orally administered or injectable compositions are merely representative. Further materials as well as processing techniques are set out in Part 5 of Reniington's Pharmaceutical Sciences, 20th Edition; 2000, Marck Publishing Company, Easton, Pennsylvania .

The compounds of this invention can also be administered in sustained release forms or from sustained release drug delivery systems. A description of representative sustained release materials can also be found in *Remington's Pharma-ceutical Sciences.*

The definition of "pharmaceutically acceptable" is meant to encompass any carrier, which does not interfere with effectiveness of the biological activity of the active ingredient and that is not toxic to the host to which it is administered. For example, for parenteral administration, IKK inhibitor may be formulated in a unit dosage form for injection in vehicles such as saline, dextrose solution, serum albumin and Ringer's solution.

For parenteral (e.g. intravenous, subcutaneous, intramuscular) administration, IKK inhibitors can be formulated as a solution, suspension, emulsion or lyophilized powder in association with a pharmaceutically acceptable parenteral vehicle (e.g. water, saline, dextrose solution) and additives that maintain isotonicity (e.g. mannitol) or chemical stability (e.g. preservatives and buffers). The formulation is sterilized by commonly used techniques.

The therapeutically effective amounts of an IKK inhibitor will be a function of many variables, including the type of inhibitor, the affinity of the inhibitor for IKK, any residual cytotoxic activity exhibited by the IKK inhibitor, the route of administration or the clinical condition of the patient.

A "therapeutically effective amount" is such that when administered, the IKK inhibitor results in inhibition of the biological activity of IKK. The dosage administered, as single or multiple doses, to an individual will vary depending upon a variety of factor, including IKK inhibitor pharmacokinetic properties, the route of administration, patient conditions and characteristics (sex, age, body weight, health, size), extent of symptoms, concurrent treatments, frequency of treatment and the effect desired. Adjustment and manipulation of established dosage ranges are well within the ability of those skilled, as well as *in vitro* and *in vivo* methods of determining the inhibition of IKK in an individual.

The IKK inhibitors are an anilinopyrimidine derivative of Formula (I)

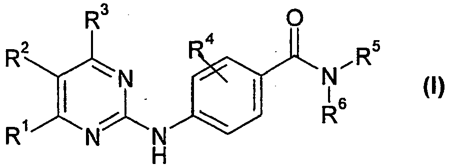

Said compounds are disclosed in WO 02/46171 (Signal Pharmaceuticals Inc.), which are described in particular for the treatment of autoimmune disorders, inflammatory diseases, cardiovascular diseases, infectious diseases, stroke or cancer.

In said compounds according to Formula (I), which include its pharmaceutically acceptable salts thereof, the substituents are defined as follows:
R¹ is either an aryl or heteroaryl optionally substituted with one to four substituents independently selected from R⁷;
R² is hydrogen;
R³ is either hydrogen or C₁₋₆ alkyl;
R⁴ is optionally one to four substituents, wherein each substituent is the same or different and is independently selected from the group consisting of halogen, hydroxy, C₁₋₆ alkyl and C₁₋₆ alkoxy;
R⁵ and R⁶ are the same or different and are independently selected from the group consisting of -R⁸, -(CH₂)ₐC(=O)R⁹, -(CH₂)ₐC(=O)OR⁹, -(CH₂)ₐC(=O)NR⁹R¹⁰, -(CH₂)ₐC(=O)NR⁹(CH₂)_{b}C(=O)R¹⁰, -(CH₂)ₐNR⁹C(=O)R¹⁰, -(CH₂)ₐNR¹¹C(=O)NR⁹R¹⁰, -(CH₂)ₐNR⁹R¹⁰, (CH₂)ₐOR⁹, -(CH₂)ₐSO_{c}R⁹ or -(CH₂)ₐSO₂NR⁹R¹⁰; and R⁵ and R⁶ taken together with the nitrogen atom to which they are attached to form a heterocycle or substituted heterocycle;
R⁷ is at each occurrence independently selected from the group consisting of halogen, hydroxy, cyano, nitro, carboxy, alkyl, alkoxy, haloalkyl, acyloxy, sulfanylalkyl, sulfinylalkyl, sulfonylalkyl, hydroxyalkyl, aryl, substituted aryl, alkylaryl, substituted alkylaryl, heterocycloalkyl, substituted heterocycloalkyl, alkylheterocycloalkyl, substituted alkylhcterocycloalkyl, -C(=O)OR⁸, -OC(=O)R⁸, - C(=O)NR⁸R⁹, -C(=O)NR⁸OR⁹, -SO_{c}R⁸, -SO_{c}NR⁸R⁹, -NR⁸SO_{c}R⁹ -NR⁸R⁹, -NR⁸C(=O)R⁹, -NR⁸C(=O)(CH₂)_{b}OR⁹, -NR⁸C(=O)(CH₂)_{b}R⁹, -O(CH₂)_{b}NR⁸R⁹ and substituted or unsubstituted heterocycloalkyl fused to substituted or unsubstituted phenyl;
R⁸, R⁹, R¹⁰ and R¹¹ arc the same or different and are at each occurrence independently selected from the group consisting of hydrogen, alkyl, substituted alkyl, aryl, substituted aryl, alkylaryl, substituted alkylaryl, heterocycloalkyl, substituted heterocycloalkyl, alkylheterocycloalkyl and substituted alkylheterocycloalkyl;
or R⁸ and R⁹ taken together with the atom or atoms to which they are attached to form a heterocycle or substituted heterocycle;
a and b are the same or different and are at each occurrence independently selected from the group consisting of 0, 1, 2, 3 or 4; and c is at each occurrence 0, 1 or 2.

In one embodiment of the invention, R is either a substituted or unsubstituted aryl or heteroaryl. When R¹ is substituted, it is substituted with one or more substituents defined below. Preferably, when substituted, R¹ is substituted with a halogen, sulfonyl or sulfonamide.

In another embodiment of the invention, R¹ is selected from the group consisting of a substituted or unsubstituted aryl, furyl, benzofuranyl, thiophenyl, benzothiophenyl, quinolinyl, pyrrolyl, indolyl, oxazolyl, benzoxazolyl, imidazolyl, benzimidazolyl, thiazolyl, benzothiazolyl, isoxazolyl, pyrazolyl, isothiazolyl, pyridazinyl, pyrimidinyl, pyrazinyl, triazinyl, cinnolinyl, phthalazinyl and quinazolinyl.

In another embodiment of the invention, R¹ is a substituted or unsubstituted aryl, preferably a substituted or unsubstituted phenyl. When R¹ is a substituted aryl, the aryl is substituted with one or more substituents defined below.

Preferably, when substituted, R¹ is substituted with a halogen, sulfonyl or sulfonamide.

In another embodiment of the invention, R⁵ and R⁶, taken together with the nitrogen atom to which they are attached form a substituted or unsubstituted nitrogen-containing non-aromatic heterocycle, preferably substituted or unsubstituted morpholinyl, substituted or unsubstituted thiomorpholinyl, substituted or unsubstituted pyrrolidinonyl, substituted or unsubstituted pyrrolidinyl, substituted or unsubstituted piperidinyl, substituted or unsubstituted homopiperidinyl, substituted or unsubstituted piperazinyl, substituted or unsubstituted homopiperazinyl, substituted or unsubstituted hydantoinyl, substituted or unsubstituted tetrahydropyridinyl, substituted or unsubstituted tetrahydropyrimidinyl, substituted or unsubstituted oxazolidinyl, substituted or unsubstituted thiazolidinyl, substituted or unsubstituted indolinyl, substituted or unsubstituted isoindolinyl, substituted or unsubstituted tetrahydroquinolinyl or substituted or unsubstituted tetrahydroisoquinolinyl.

When R⁵ and R⁶, taken together with the nitrogen atom to which they are attached form a substituted or unsubstituted piperazinyl, a substituted or unsubstituted piperadinyl or a substituted or unsubstituted morpholinyl, the substituted piperazinyl, substituted piperadinyl or substituted morpholinyl is substituted with one or more substituents defined below.

Preferably, when substituted, the substituent is alkyl, amino, alkylamino, alkylether, acyl, pyrrolidinyl or piperidinyl:

In one embodiment of the invention, R², R³ and R⁴ are hydrogen, and the compounds of this invention has the following Formula (II):

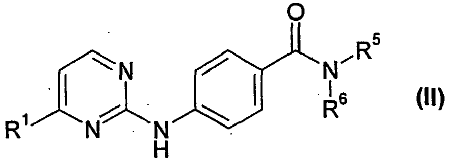

In a more specific embodiment of the invention, R¹ is a phenyl optionally substituted with R⁷, and having the following Formula (III):

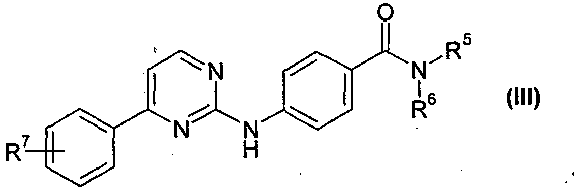

In still a further embodiment of the invention, R⁷ is at the para position of the phenyl ring, as represented by the following Formula (IV):

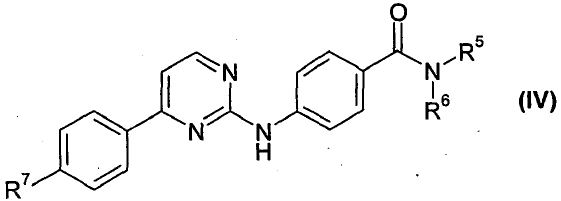

In still a further embodiment of the invention, in the anilinopyrimidine derivatives is 1-(4-{4-[4-(4-Chloro-phenyl)-pyrimidin-2-ylamino]-benzoyl}-piperazin-1-yl)-ethanone.

The syntheses of compounds of Formulae (I)-(IV) is described in detail in WO 02/46171 (Signal Pharmaceuticals Inc.).

Another IKK inhibitor is a compound as shown in Formula (V) (Burke et al. (2003)):

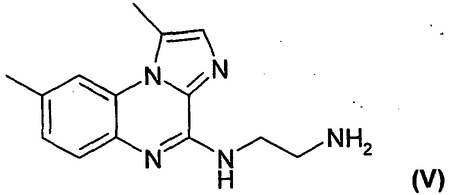

Another IKK inhibitor is a compound as shown in Formula (VI) (Coghlan et al (2003):

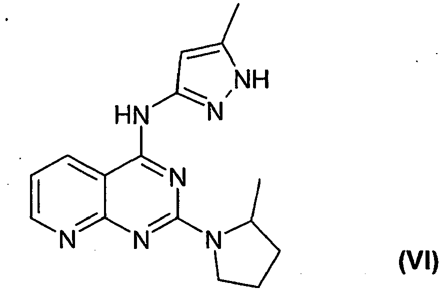

Another IKK inhibitor is a compound as shown in Formula (VII) (Hideshima et al. (2002):

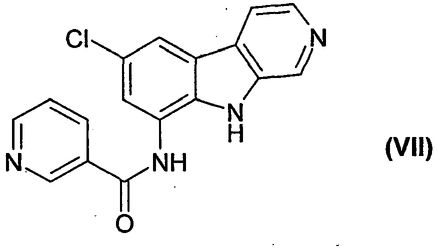

Another IKK inhibitor is a compound as shown in Formula (VIII) (.WO 02/44153, Bayer):

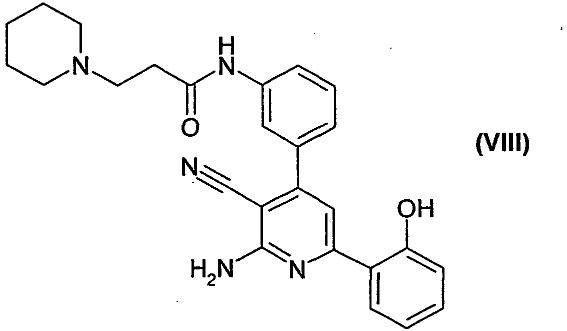

Another IKK inhibitor is a compound as shown in Formula (IX) (Kishore et al. (2003):

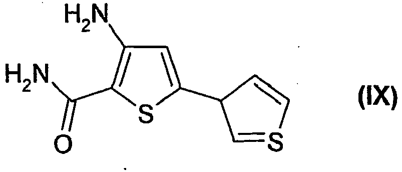

Another IKK inhibitor is a compound as shown in Formula (X) (Podolin et al. (2005) J. Pharmacol. Exp. Ther.):

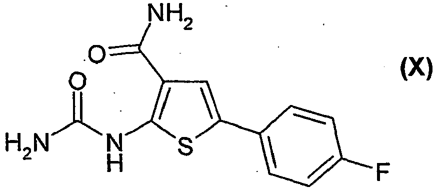

Another IKK inhibitor is a compound as shown in Formula (XI) (Baxter et al. (2004):

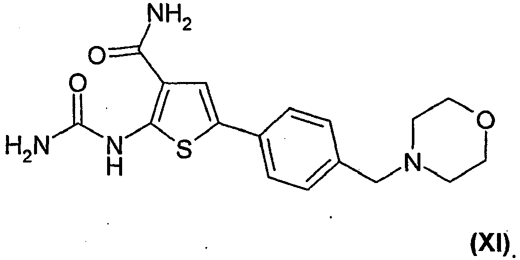

Another IKK inhibitor is a compound as shown in Formula (XII) (WO 04/022553, Aventis Pharma):

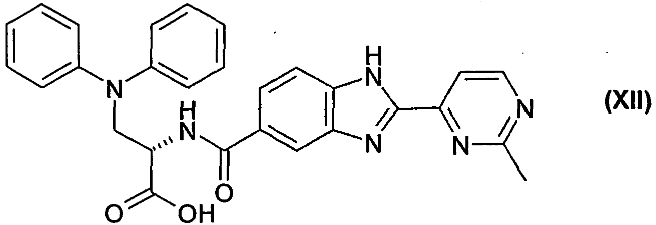

Compounds (V) to (XII) do not form part of the present invention.

The present invention will now be illustrated by the example .

### Examples

### Example 1: Models for endometriosis

The effect of IKK inhibitors was evaluated in both in vitro and in vivo models of endometriosis.

### Example 1.1: Cytokine Secretion

For in vitro studies, human endometriotic cells (12Z) were used (Zeitvogel et al 2001). These cells secrete cytokines in response to TNFα that have previously been reported to be elevated in the peritoneal fluid of endometriotic patients. Endometriotic 12Z cells were stimulated with TNFα for 24h and the culture supernatant was measured for the presence of GMCSF, IL-6 and IL-8. These cytokines were quantitated by Meso Scale Discovery technology. This is a multiplex assay platform, employing sandwich type ELISA. In each 96-well, antibodies for IL-6, IL-8 and GMCSF were spotted to measure all three cytokines in the same well. All three cytokines are increased in response to different concentrations of TNFα in 12Z cells.

Five different IKK inhibitors were used to inhibit cytokine expression. N-(6-Chloro-9H-beta-carbolin-8-yl)-nicotinamide (an inhibitor of formula VII), {4-[4-(4-Chloro-phenyl)-pyrimidin-2-ylamino]-phenyl}-piperazin-1-yl-methanone, {4-[4-(4-Chloro-phenyl)-pyrimidin-2-ylamino]-phenyl}-[4-(2-hydroxy-ethyl)-piperazin-1-yl]-methanone, {4-[4-(4-Chloro-phenyl)-pyrimidin-2-ylamino]-phenyl}-[4-(2-ethoxy-ethyl)-piperazin-1-yl]-methanone and 1-(4-{4-[4-(4-Chloro-phenyl)-pyrimidin-2-ylamino]-benzoyl}-piperazin-1-yl)-ethanone (all inhibitors of formula I) were tested. In the presence of a fixed concentration of TNFα (15 ng/ml), all IKK inhibitors dose-dependently blocked cytokine secretion. Inhibitors by themselves did not cause any significant effect on the cytokines. Thus, IKK inhibitors are capable of blocking TNF-α induced cytokine secretion by human endometriotic cells. By blocking the IKK pathway, inflammatory cytokines associated with endometriosis can be specifically inhibited and thus these IKK inhibitors are useful for treating endometriosis.

### Example 1.2: Nude Mouse Model

Human endometrial tissue was injected in ovarectomized nude mice to establish the disease (Bruner-Tran et al 2002). In brief, endometrial biopsies obtained from normal volunteers or from endometriotic patients were cut into small pieces and cultured in the presence of estradiol for 24h. Treated tissues, were injected either subcutaneously or intraperitoneally into ovarectomized nude mice with estradiol implant. Within 2-4 days of injection, ectopic endometriotic lesions develop in animals. Treatment with either progesterone or IKK inhibitor-5 (1-(4-{4-[4-(4-Chloro-phenyl)-pyrimidin-2-ylamino]-benzoyl}-piperazin-1-yl)-ethanone) was started 10 days following the injection of tissue. The compound was administered at a dose of 10mg/kg and 30mg/kg/animal for 15 days. Earlier work using this model has established that progesterone treatment prevents disease progression, hence this was used as control. Following the completion of treatment, animals were sacrificed, lesions developed from the transplanted tissue found in both subcutaneous and intraperitoneal sites, were measured (both size and number).

Table 1 below, illustrates the results of studies carried out in nude mice. The IKK inhibitor at a dose of 10mg/kg and 30mg/kg was effective in regressing the established disease by 125% and 100% respectively compared to progesterone treatment. The mean lesion size was also reduced by 90% and 75% respectively by the treatment. These results are significant, since the model measures the growth/regression of human endometrial tissue and thus has a direct relevance for treating the human disease. The IKK inhibitor treatment had no effect on the uterine weight and size of the animals.

**Table 1: Effect of IKK inhibitor-5 on regression of endometriotic lesions in the nude mouse xenograft model.**

| **Treatment** | **Lesion (% Progesterone)** Compared to progesterone treated group | **Lesion size** |
|---|---|---|
| IKK inhibitor-5 10 mg/kg x 15 days | 125 % Decrease | 90 % Decrease |
| IKK inhibitor-5 30 mg/kg x 15 days | 100 % Decrease | 75% Decrease |

### Reference List

Burke et al. (2003) J. Biol Chem, 278, 1450-1456
Coghlan et al. (2003) Inflam. Res. 52, 2-5
D'Hooghe et al. ASRM (2001)
Dawood et al. (1993) Int. J. Gynaecol.Obstet. 40 (Suppl.), 29-42
Giudice et al. (2004) Lancet 364, 1789-99
Hideshima et al. (2002) J- Biol. Chem. 19, 16639-47
Kishore et al. (2003) J. Biol. Chem. 35, 32861-71
Kyama et al. (2003) Reprod Biol Endocrinol.1, 123
Mercurio et al. (1997) (1997) Science 278, 860-866)
Podolin et al. (2005) J. Pharmacol. Exp. Ther.
Waller et al. (1993) Fertil.Steril. 59, 511-515
Woronicz et al. (1997) Science 278, 866-869
Zeitvogel et al. (2001) Am. J Pathol. 159 1839-52
EP 160,699
EP 211,894
EP 322,438
WO 02/44153
WO 02146171
WO 04/022553

## Claims

1. An IKK inhibitor according to Formula (I) 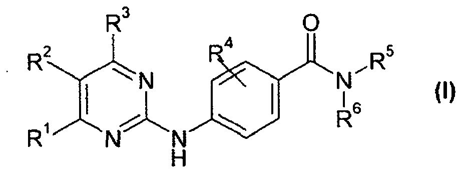 as well as its pharmaceutically acceptable salts, wherein
R¹ is either aryl or heteroaryl optionally substituted with one to four substituents independently selected from R⁷;
R² is hydrogen;
R³ is either hydrogen or C₁-C₆alkyl;
R⁴ is optionally present and denotes one to four substituents, wherein each substituent is the same or different and is independently selected from the group consisting of halogen, hydroxy, C₁ₗ-C₆all<yl and C₁-C₆alkoxy;
R⁵ and R⁶ are the same or different and are independently selected from the group consisting of -R⁸, -(CH₂)ₐC(=O)R⁹, -(CH₂)aC(=O)OR⁹, -(CH₂)ₐC(=O)NR⁹R¹⁰, - (CH₂)ₐC(=O)NR⁹(CH₂)_{b}C(=O)R¹⁰, -(CH₂)ₐNR⁹C(=O)R¹⁰, -(CH₂)ₐNR¹¹C(=O)NR⁹R¹⁰, -(CH₂)ₐNR⁹R¹⁰, (CH₂)ₐOR⁹, -(CH₂)ₐSO_{c}R⁹ and -(CH₂)ₐSO₂NR⁹R¹⁰;
or R⁵ and R⁶ taken together with the nitrogen atom to which they are attached to form an optionally substituted heterocycle;
R⁷ is at each occurrence independently selected from the group consisting of halogen, hydroxy, cyano, nitro, carboxy, alkyl, alkoxy, haloalkyl, acyloxy, sulfanylalkyl, sulfinylalkyl, sulfonylalkyl, hydroxyalkyl, aryl, substituted aryl, alkylaryl, substituted alkylaryl, heterocycloalkyl, substituted heterocycloalkyl, alkylheterocycloalkyl, substituted alkylheterocycloalkyl, -C(=O)OR⁸, -OC(=O)R⁸, -C(=O)NR⁸R⁹, - C(=O)NR⁸OR⁹, -SO_{c}R⁸, -SO_{c}NR⁸R⁹, -NR⁸SO_{c}R⁹ -NR⁸R⁹, -NR⁸C(=O)R⁹, - NR⁸C(=O)(CH₂)_{b}OR⁹, -NR⁸C(=O)(CH₂)_{b}R⁹, -O(CH₂)_{b}NR⁸R⁹ and heterocycloalkyl fused to phenyl;
R⁸, R⁹, R¹⁰ and R¹¹ are the same or different and are at each occurrence independently selected from the group consisting of hydrogen, alkyl, substituted alkyl, aryl, substituted aryl, alkylaryl, substituted alkylaryl, heterocycloalkyl, substituted heterocycloalkyl, alkylheterocycloalkyl and substituted alkylheterocycloalkyl;
or R⁸ and R⁹ taken together with the atom or atoms to which they are attached to form an optionally substituted heterocycle;
a and b are the same or different and are at each occurrence independently selected from the group consisting of 0, 1, 2, 3 and 4; and
c is at each occurrence 0, 1 or 2.
for use in a method for treating and/or preventing endometriosis in an individual.

2. An IKK inhibitor for use according to claim 1 in combination with a hormonal suppressor.

3. An IKK inhibitor for use according to claim 1 or 2, wherein said hormonal suppressor is selected from the group consisting of a GnRH antagonist, GnRH agonist, aromatase inhibitor, progesterone receptor modulator and an estrogen receptor modulator.

4. An IKK inhibitor for use for use according to any one of claims 1 to 3, wherein said IKK inhibitor is administered alone or in combination with drugs for the treatment of endometriosis-related infertility.

5. An IKK inhibitor for use according to claim 1, wherein R⁵ and R⁶, taken together with the nitrogen atom to which they are attached form an optionally substituted nitrogen-containing non-aromatic heterocycle.

6. An IKK inhibitor for use according to claim 1 or 5, wherein the nitrogen-containing non-aromatic heterocycle is selected from the group consisting of morpholinyl, thiomorpholinyl, pyrrolidinonyl, pyrrolidinyl, piperidinyl, homopiperidinyl, piperazinyl, homopiperazinyl, hydantoinyl, tetrahydropyriodinyl, tetrahydropyrimidinyl, oxazolidinyl, thiazolidinyl, indolinyl, isoindolinyl, tetrahydroquinolinyl and tetrahydroisoquinolinyl.

7. An IKK inhibitor for use according to any one of claims 1 or 5 to 6, wherein R¹ is either aryl or heteroaryl.

8. An IKK inhibitor for use according to any one of claims 1 or 5 to 7, wherein R¹ is selected from the group consisting of aryl, furyl, benzofuranyl, thiophenyl, benzothiophenyl, quinolinyl, pyrrolyl, indolyl, oxazolyl, benzoxazolyl, imidazolyl, benzimidazolyl, thiazolyl, benzothiazolyl, isoxazolyl, pyrazolyl, isothiazolyl, pyridazinyl, pyrimidinyl, pyrazinyl, triazinyl, cinnolinyl, phthalazinyl and quinazolinyl.

9. An IKK inhibitor for use according to any one of claims 1 or 5 to 8, wherein R¹ is phenyl.

10. An IKK inhibitor for use according to any one of claims 5 to 9, wherein the nitrogen-containing heterocycle is piperazinyl.

11. An IKK inhibitor for use according to any one of claims 5 to 9, wherein the nitrogen-containing heterocycle is piperidinyl.

12. An IKK inhibitor for use according to any one of claims 5 to 9, wherein the nitrogen-containing heterocycle is morpholinyl.

13. An IKK inhibitor for use according to any one of claims 1 to 4, wherein said IKK inhibitor is a compound according to Formula (II): 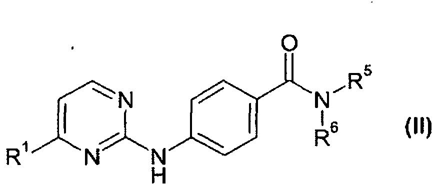 as well as pharmaceutically acceptable salts, wherein
R¹ is aryl or heteroaryl optionally substituted with one to four substituents independently selected from R⁷;
R⁵ and R⁶ are the same or different and are independently selected from the group consisting of -R⁸, -(CH₂)ₐC(=O)R⁹ -(CH₂)ₐC(=O)OR⁹, -(CH₂)ₐC(=O)NR⁹R¹⁰, -(CH₂)ₐC(=O)NR⁹(CH₂)_{b}C(=O)R¹⁰, -(CH₂)ₐNR⁹C(=O)R¹⁰, -(CH₂)ₐNR¹¹C(=O)NR⁹R¹⁰, -(CH₂)ₐNR⁹R¹⁰, (CH₂)ₐOR⁹, -(CH₂)ₐSO_{c}R⁹ and -(CH₂)ₐSO₂NR⁹R¹⁰;
or R⁵ and R⁶ taken together with the nitrogen atom to which they are attached to form a heterocycle or substituted heterocycle;
R⁷ is at each occurrence independently selected from the group consisting of halogen, hydroxy, cyano, nitro, carboxy, alkyl, alkoxy, haloalkyl, acyloxy, sulfanylalkyl, sulfinylalkyl, sulfonylalkyl, hydroxyalkyl, aryl, substituted aryl, alkylaryl, substituted alkylaryl, heterocycloalkyl, substituted heterocycloalkyl, alkylheterocycloalkyl, substituted alkylheterocycloalkyl, -C(=O)OR⁸, -OC(=O)R⁸, -C(=O)NR⁸R⁹, -C(=O)NR⁸OR⁹, -SO_{c}R⁸, -SO_{c}NR⁸R⁹, -NR⁸SO_{c}R⁹ -NR⁸R⁹, -NR⁸C(=O)R⁹, -NR⁸C(=O)(CH₂)_{b}OR⁹, -NR⁸C(=O)(CH₂)_{b}R⁹, -O(CH₂)_{b}NR⁸R⁹ and heterocycloalkyl fused to phenyl;
R⁸, R⁹, R¹⁰ and R¹¹ are the same or different and are at each occurrence independently selected from the group consisting of hydrogen, alkyl, substituted alkyl, aryl, substituted aryl, alkylaryl, substituted alkylaryl, heterocycloalkyl, substituted heterocycloalkyl, alkylheterocycloalkyl and substituted alkylheterocycloalkyl;
or R⁸ and R⁹ taken together with the atom or atoms to which they are attached to form an optionally substituted heterocycle;
a and b are the same or different and are at each occurrence independently selected from the group consisting of 0, 1, 2, 3 and 4; and
c is at each occurrence 0, 1 or 2.

14. An IKK inhibitor for use according to claim 13, wherein R⁵ and R⁶, taken together with the nitrogen atom to which they are attached form an optionally substituted nitrogen-containing non-aromatic heterocycle.

15. An IKK inhibitor for use according to claim 13 or 14, wherein the nitrogen-containing non-aromatic heterocycle is selected from the group consisting of morpholinyl, thiomorpholinyl, pyrrolidinonyl, pyrrolidinyl, piperidinyl, homopiperidinyl, piperazinyl, homopiperazinyl, hydantoinyl, tetrahydropyridinyl, tetrahydropyrimidinyl, oxazolidinyl, thiazolidinyl, indolinyl, isoindolinyl, tetrahydroquinolinyl and tetrahydroisoquinolinyl.

16. An IKK inhibitor for use according to any one of claims 13 to 15, wherein R¹ is either aryl or heteroaryl.

17. An IKK inhibitor for use according to any one of claims 13 to 16, wherein R¹ is selected from the group consisting of aryl, furyl, benzofuranyl, thiophenyl, benzothiophenyl, quinolinyl, pyrrolyl, indolyl, oxazolyl, benzoxazolyl, imidazolyl, benzimidazolyl, thiazolyl, benzothiazolyl, isoxazolyl, pyrazolyl, isothiazolyl, pyridazinyl, pyrimidinyl, pyrazinyl, triazinyl, cinnolinyl, phthalazinyl and quinazolinyl.

18. An IKK inhibitor for use according to any one of claims 13 to 17, wherein R¹ is phenyl.

19. An IKK inhibitor for use according to any one of claims 14 to 18, wherein the nitrogen-containing heterocycle is piperazinyl.

20. An IKK inhibitor for use according to any one of claims 14 to 18, wherein the nitrogen-containing heterocycle is piperidinyl.

21. An IKK inhibitor for use according to any one of claims 14 to 18, wherein the nitrogen-containing heterocycle is morpholinyl.

22. An IKK inhibitor for use according to any one of claims 1 to 4, wherein said IKK inhibitor is a compound according to Formula (III): 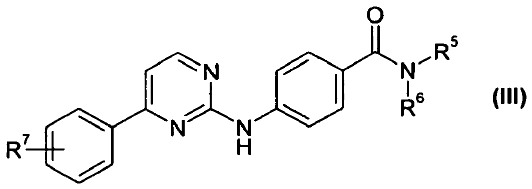 wherein R⁵, R⁶, R⁷, R⁸, R⁹, R¹⁰ and R¹¹ are defined as in claims 13 to 21.

23. An IKK inhibitor for use according to any one of claims 1 to 4, wherein said IKK inhibitor is a compound according to Formula (IV): 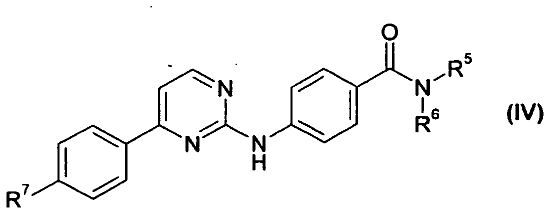 wherein R⁵, R⁶, R⁷, R⁸, R⁹, R¹⁰ and R¹¹ are defined as in claims 13 to 21.

24. An IKK inhibitor for use according to any one of claims 1 to 4, wherein said IKK inhibitor is 1-(4-{4-[4-(4-Chloro-phenyl)-pyrimidin-2-ylamino]-benzoyl}-piperazin-1-yl)-ethanone.

25. A pharmaceutical composition comprising an IKK inhibitor of Formula (I) as defined in claim 1, a hormonal suppressor, and a pharmaceutically acceptable excipient for use in treating and/or preventing endometriosis in an individual.

26. A pharmaceutical composition comprising an IKK inibitor of Formula (I) as defined in claim 1, and an hormonal suppressor selected from the group consisting of a GnRH antagonist, aromatase inhibitor, and progesterone receptor modulator.

27. The pharmaceutical composition according to claim 25 or 26, wherein said IKK inhibitor is 1-(4-{4-[4-(4-Chloro-phenyl)-pyrimidin-2-ylamino]-benzoyl}-piperazin-1-yl)-ethanone.

## Patentansprüche

1. IKK-Hemmer gemäß Formal (I) 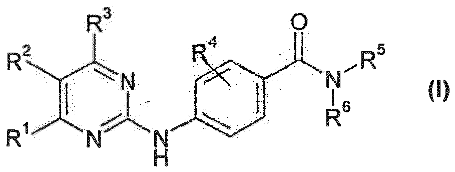 sowie dessen pharmazeutisch zulässigen Salze, worin
R¹ entweder ein Aryl oder Heteroaryl ist, gegebenenfalls substituiert mit einem bis vier Substituenten, die unabhängig voneinander ausgewählt sind aus R⁷;
R² Wasserstoff ist;
R³ entweder Wasserstoff oder C₁₋₆-Alkyl ist;
R⁴ gegebenenfalls vorhanden ist und ein bis vier Substituenten bezeichnet, worin jeder Substituent der selbe oder ein unterschiedlicher ist und unabhängig voneinander ausgewählt ist aus der Gruppe bestehende aus Halogen, Hydroxy, C₁-C₆-Alkyl und C₁-C₆-Alkoxy;
R⁵ und R⁶ gleich oder unterschiedlich sind und unabhängig voneinander ausgewählt sind aus der Gruppe bestehend aus -R⁸ , -(CH₂)ₐC(=O)R⁹, -(CH₂)ₐC (=O)OR⁹ , -(CH₂)ₐC (=O)NR⁹R¹⁰, -(CH₂)ₐC(=O)NR⁹(CH₂)_{b}C(=O)R¹⁰, -(CH₂)ₐNR⁹C(=O)R¹⁰, - (CH₂)ₐNR¹¹C(=O)NR⁹R¹⁰, -(CH₂)ₐNR⁹R¹⁰, (CH₂)ₐOR⁹ -(CH₂)ₐSO_{c}R⁹ und - -(CH₂)ₐSO₂NR⁹R¹⁰;
oder R⁵ und R⁶ zusammengenommen mit dem Stickstoffatom, an welches sie angebracht sind, einen gegebenenfalls substituierten Heterocyclus bilden;
R⁷ bei jedem Vorkommen unabhängig ausgewählt ist aus der Gruppe bestehend aus Halogen, Hydroxy, Cyano, Nitro, Carboxy, Alkyl, Alkoxy, Haloalkyl, Acyloxy, Sulfanylalkyl, Sulfinylalkyl, Sulfonylalkyl, Hydroxyalkyl, Aryl, substituiertes Aryl, Alkylaryl, substituiertes Alkylaryl, Heterocycloalkyl, substituiertes Heterocycloalkyl, Alkylheterocycloalkyl, substituiertes Alkylheterocycloalkyl, -C(=O)OR⁸, -OC(=O)R⁸, - C(=O)NR⁸R⁹ -C(=O)NR⁸OR⁹, -SO_{c}R⁸, -SO_{c}NR⁸R⁹ -NR⁸SO_{c}R⁹ -NR⁸R⁹, -NR⁸C(=O)R⁹ - NR^{g}C(=O)(CH₂)_{b}OR⁹, -NR⁸C(=O)(CH₂)_{b}R⁹, -O(CH₂)_{b}NR⁸R⁹ und an Phenyl fusioniertes Heterocyctoalkyl;
R⁸, R⁹, R¹⁰ und R¹¹ sind gleich oder unterschiedlich und sie sind bei jedem Vorkommen unabhängig voneinander ausgewählt aus der Gruppe bestehend aus Wasserstoff, Alkyl, substituiertes Alkyl, Aryl, substituiertes Aryl, Alkylaryl, substituiertes Alkylaryl, Heterocycloalkyl, substituiertes Heterocycloalkyl, Alkylheterocycloalkyl und substituiertes Alkylheterocycloalkyl;
oder R⁸ und R⁹ zusammengenommen mit dem Atom oder den Atomen, an welche(s) sie angebracht sind, um einen gegebenenfalls substituierten Heterocyclus zu bilden;
a und b gleich oder unterschiedlich sind und bei jedem Vorkommen unabhängig voneinander ausgewählt sind aus der Gruppe bestehende aus 0, 1, 2, 3 und 4; und
c bei jedem Vorkommen 0, 1 oder 2 ist
für die Verwendung in einem Verfahren zur Behandlung und/oder Vorbeugung von Endomtriose in einem Individuum.

2. IKK-Hemmer zur Verwendung gemäß Anspruch 1, in Kombination mit einem Hormonsuppressor.

3. IKK-Hemmer zur Verwendung gemäß Anspruch 1 oder 2, wobei der Hormonsuppressor ausgewählt ist aus der Gruppe bestehend aus einem GnRH-Antagonisten, GnRH-Agonisten, Aromatasehemmer, Progesteronrezeptor-Modulator und Östrogenrezeptor-Modulator.

4. IKK-Hemmer zur Verwendung gemäß einem der Ansprüche 1 bis 3, worin der besagte IKK-Hemmer alleine oder in Kombination mit Medikamenten für die Behandlung von mit Endometriose in Verbindung stehender Unfruchtbarkeit verabreicht wird.

5. IKK-Hemmer zur Verwendung gemäß Anspruch 1, worin R⁵ und R⁶, zusammengenommen mit dem Stickstoffatom, an welches sie angebracht sind, einen gegebenenfalls substituierten, Stickstoff enthaltenden, nicht-aromatischen Heterocyclus bilden.

6. IKK-Hemmer zur Verwendung gemäß Anspruch 1 oder 5, worin der Stickstoff enthaltende, nicht-aromatische Heterocyclus ausgewählt ist aus der Gruppe bestehend aus Morpholinyl, Thiomorpholinyl, Pyrrolidinonyl, Pyrrolidinyl, Piperidinyl, Homopiperidinyl, Piperazinyl, Homopiperazinyl, Hydantoinyl, Tetrahydropyridinyl, Tetrahydropyrimidinyl, Oxazolidinyl, Thiazolidinyl, Indolinyl, Isoindolinyl, Tetrahydroquinolinyl und Tetrahydroisoquinolinyl.

7. IKK-Hemmer zur Verwendung gemäß einem der Ansprüche 1 oder 5 bis 6, worin R¹ entweder Aryl oder Heteroaryl ist.

8. IKK-Hemmer zur Verwendung gemäß einem der Ansprüche 1 oder 5 bis 7, worin R¹ ausgewählt ist aus der Gruppe bestehend aus Aryl, Furyl, Benzofuranyl, Thiophenyl, Benzothiophenyl, Quinolinyl, Pyrrolyl, Indolyl, Oxazolyl, Benzoxazolyl, Imidazolyl, Benzimidazolyl, Thiazolyl, Benzothiazolyl, Isoxazolyl, Pyrazolyl, Isothiazolyl, Pyridazinyl, Pyrimidinyl, Pyrazinyl, Triazinyl, Cinnolinyl, Phthalazinyl und Quinazolinyl.

9. IKK-Hemmer zur Verwendung gemäß einem der Ansprüche 1 oder 5 bis 8, worin R¹ Phenyl ist.

10. IKK-Hemmer zur Verwendung gemäß einem der Ansprüche 5 bis 9, worin der Stickstoff enthaltende Heterocyclus Piperazinyl ist.

11. IKK-Hemmer zur Verwendung gemäß einem der Ansprüche 5 bis 9, worin der Stickstoff enthaltende Heterocyclus Piperidinyl ist.

12. IKK-Hemmer zur Verwendung gemäß einem der Ansprüche 5 bis 9, worin der Stickstoff enthaltende Heterocyclus Morpholinyl ist.

13. IKK-Hemmer für die Verwendung gemäß einem der Ansprüche 1 bis 4, worin der besagte IKK-Hemmer eine Verbindung gemäß Formel (II) ist: 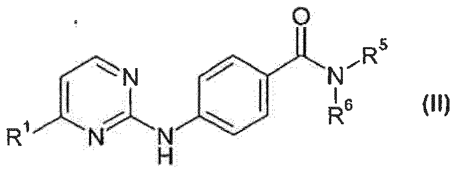 sowie dessen pharmazeutisch zulässigen Salze, worin
R¹ Aryl oder Heteroaryl ist, gegebenenfalls substituiert mit einem bis vier Substituenten, die unabhängig voneinander ausgewählt sind aus R⁷;
R⁵ und R⁶ gleich oder unterschiedlich sind und unabhängig voneinander ausgewählt sind aus der Gruppe bestehend aus -R⁸ , -(CH₂)ₐC(=O)R⁹ , -(CH₂)ₐC (=O)OR⁹ , -(CH₂)ₐC (=O)NR⁹R¹⁰, -(CH₂)ₐC(=O)NR⁹(CH₂)_{b}C(=O)R¹⁰, -(CH₂)ₐNR⁹C(=O)R¹⁰, - (CH₂)ₐNR¹¹C(=O)NR⁹R¹⁰, -(CH₂)ₐNR⁹R¹⁰, (CH₂)ₐOR⁹, (CH₂)ₐSO_{c}R⁹ und - -(CH₂)ₐSO₂NR⁹R¹⁰;
oder R⁵ und R⁶ zusammengenommen mit dem Stickstoffatom, an welches sie angebracht sind, einen Hetcrocyclus oder einen substituierten Heterocyclus bilden;
R⁷ bei jedem Vorkommen unabhängig ausgewählt ist aus der Gruppe bestehend aus Halogen, Hydroxy, Cyano, Nitro, Carboxy, Alkyl, Alkoxy, Haloalkyl, Acyloxy, Sulfanylalkyl, Sulfinylalkyl, Sulfonylalkyl, Hydroxyalkyl, Aryl, substituiertes Aryl, Alkylaryl, substituiertes Alkylaryl, Heterocycloalkyl, substituiertes eterocycloalkyl, Alkylheterocycloalkyl, substituiertes Alkylheterocycloalkyl, -C(=O)OR⁸, -OC(=O)R⁸, - C(=O)NR⁸R⁹, -C(=O)NR⁸OR⁹, -SOCR⁸, -SO_{c}NR⁸R⁹, -NR⁸SO_{c}R⁹, -NR⁸R⁹, -NR^{g}C(=O)R⁸, - NR⁸C(=O)(CH₂)_{b}OR⁹, -NR⁸C(=O)(CH₂)_{b}R⁹, -O(CH₂)_{b}NR⁸R⁹ und an Phenyl fusioniertes Heterocyctoalkyl;
R⁸, R⁹, R¹⁰ und R¹¹ sind gleich oder unterschiedlich und sie sind bei jedem Vorkommen unabhängig voneinander ausgewählt aus der Gruppe bestehend aus Wasserstoff, Alkyl, substituiertes Alkyl, Aryl, substituiertes Aryl, Alkylaryl, substituiertes Alkylaryl, Heterocycloalkyl, substituiertes Heterocycloalkyl, Alkylheterocycloalkyl und substituiertes Alkylheterocycloalkyl;
oder R⁸ und R⁹ zusammengenommen mit dem Atom oder den Atomen, an welche(s) sie angebracht sind, um einen gegebenenfalls substituierten Heterocyclus zu bilden;
a und b gleich oder unterschiedlich sind und bei jedem Vorkommen unabhängig voneinander ausgewählt sind aus der Gruppe bestehende aus 0, 1, 2, 3 und 4; und
c bei jedem Vorkommen 0, 1 oder 2 ist.

14. IKK-Hemmer zur Verwendung gemäß Anspruch 13, worin R⁵ und R⁶, zusammengenommen mit dem Stickstoffatom, an welches sie angebracht sind, einen gegebenenfalls substituierten, Stickstoff enthaltenden, nicht-aromatischen Heterocyclus bilden.

15. IKK-Hemmer zur Verwendung gemäß Anspruch 13 oder 14, worin der Stickstoff enthaltende, nicht-aromatische Heterocyclus ausgewählt ist aus der Gruppe bestehend aus Morpholinyl, Thiomorpholinyl, Pyrrolidinonyl, Pyrrolidinyl, Piperidinyl, Homopiperidinyl, Piperazinyl, Homopiperazinyl, Hydantoinyl, Tetrahydropyridinyl, Tetrahydropyrimidinyl, Oxazolidinyl, Thiazolidinyl, Indolinyl, Isoindolinyl, Tetrahydroquinolinyl und Tetrahydroisoquinolinyl.

16. IKK-Hemmer zur Verwendung gemäß einem der Ansprüche 13 bis 15, worin R¹ entweder Aryl oder Heteroaryl ist.

17. IKK-Hemmer zur Verwendung gemäß einem der Ansprüche 13 bis 16, worin R¹ ausgewählt ist aus der Gruppe bestehend aus Aryl, Furyl, Benzofuranyl, Thiophenyl, Benzothiophenyl, Quinolinyl, Pyrrolyl, Indolyl, Oxazolyl, Benzoxazolyl, Imidazolyl, Benzimidazolyl, Thiazolyl, Benzothiazolyl, Isoxazolyl, Pyrazolyl, Isothiazolyl, Pyridazinyl, Pyrimidinyl, Pyrazinyl, Triazinyl, Cinnolinyl, Phthalazinyl und Quinazolinyl.

18. IKK-Hemmer zur Verwendung gemäß einem der Ansprüche 13 bis 17, worin R¹ Phenyl ist.

19. IKK-Hemmer zur Verwendung gemäß einem der Ansprüche 14 bis 18, worin der Stickstoff enthaltende Heterocyclus Piperazinyl ist.

20. IKK-Hemmer zur Verwendung gemäß einem der Ansprüche 14 bis 18, worin der Stickstoff enthaltende Heterocyclus Piperidinyl ist.

21. IKK-Hemmer zur Verwendung gemäß einem der Ansprüche 14 bis 18, worin der Stickstoff enthaltende Heterocyclus Morpholinyl ist.

22. IKK-Hemmer für die Verwendung gemäß einem der Ansprüche 1 bis 4, worin der besagte IKK-Hemmer eine Verbindung gemäß Formel (III) ist: 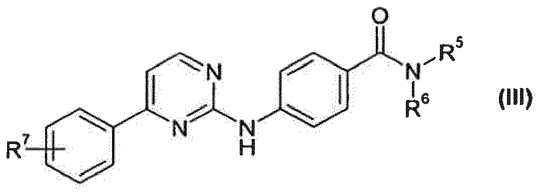 R⁵, R⁶, R⁷, R⁸, R⁹, R¹⁰ und R¹¹, so definiert sind, wie in den Ansprüche 13 bis 21.

23. IKK-Hemmer für die Verwendung gemäß einem der Ansprüche 1 bis 4, worin der besagte IKK-Hemmer eine Verbindung gemäß Formel (IV) ist: 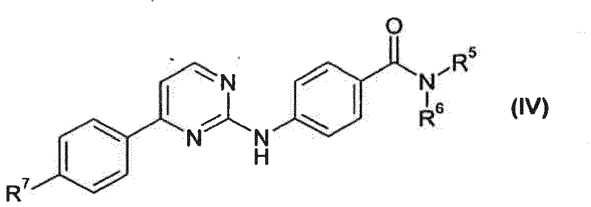 worin R⁵, R⁶, R⁷, R⁸, R⁹, R¹⁰ und R¹¹ so definiert sind, wie in den Ansprüche 13 bis 21.

24. IKK-Hemmer für die Verwendung gemäß einem der Ansprüche 1 bis 4, worin der besagte IKK-Hemmer 1-(4-{4-[4-(4-Chloro-phenyl)-pyrimidin-2-ylamino]-benzoyl}-piperazin-1-yl)-ethanon ist.

25. Pharmazeutische Zusammensetzung, welche einen IKK-Hemmer der Formel (I), wie er in Anspruch 1 definiert ist, einen Hormonsuppressor und einen pharmazeutisch zulässigen Hilfsstoff aufweist, für die Verwendung bei der Behandlung und/oder Vorbeugung von Endometriose in einem Individuum.

26. Pharmazeutische Zusammensetzung, welche einen IKK-Hemmer der Formel (I), wie er in Anspruch 1 definiert ist, und einen Hormonsuppressor, der ausgewählt ist aus der Gruppe bestehend aus einem GnRH-Antagonisten, Aromatasehemmer, und Progesteronrezeptor-Modulator, aufweist.

27. Pharmazeutische Zusammensetzung gemäß Anspruch 25 oder 26, worin der besagte IKK-Hemmer 1-(4-{4-[4-(4-Chloro-phenyl)-pyrimidin-2-ylamino]-benzoyl}-piperazin-1-yl)-ethanon ist.

## Revendications

1. Inhibiteur de kinase IKK, de formule (I) : 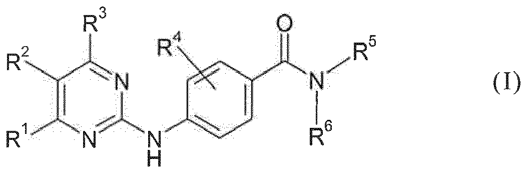 ainsi que ses sels pharmacologiquement admissibles,
dans laquelle formule
- R¹ représente un groupe aryle ou hétéroaryle, en option porteur de 1 à 4 substituant(s) choisi(s) indépendamment parmi ceux qui sont symbolisés par R⁷ ;
- R² représente un atome d'hydrogéne ;
- R³ représente un atome d'hydrogène ou un groupe alkyle en C₁₋₆ ;
- R⁴ représente 1 à 4 substituant(s) qui n'est ou ne sont présent(s) qu'en option, qui peuvent être identiques ou différents les uns des autres, et qui est ou sont indépendamment choisi(s) dans l'ensemble formé par les atomes d'halogène et les groupes hydroxyle, alkyle en C₁₋₆ et alcoxy en C₁₋₆ ;
- R⁵ et R⁶ représentent des entités identiques ou différentes, choisies indépendamment dans l'ensemble formé par les entités symbolisées par -R⁸, -(CH₂)ₐC(=O)R⁹, -(CH₂)ₐC(=O)OR⁹, -(CH₂)ₐC(=O)NR⁹R¹⁰, -(CH₂)ₐC(=O)NR⁹(CH₂)_{b}C(=O)R¹⁰, -(CH₂)ₐNR⁹C(=O)R¹⁰, -(CH₂)ₐNR¹¹C(=O)NR⁹R¹⁰, -(CH₂)ₐNR⁹R¹⁰, -(CH₂)ₐOR⁹, -(CH₂)ₐSO_{c}R⁹, et -(CH₂)ₐSO₂NR⁹R¹⁰,
ou bien R⁵ et R⁶ représentent des entités qui, conjointement avec l'atome d'azote auquel elles sont liées, forment un hétérocycle, en option porteur d'un ou de substituant(s) ;
- R⁷ représente une entité choisie, indépendamment en chaque occurrence, dans l'ensemble formé par les atomes d'halogène, les groupes hydroxyle, cyano, nitro, carboxyle, alkyle, alcoxy, halogéno-alkyle, acyloxy, sulfanyl-alkyle, sulfinyl-alkyle, sulfonyl-alkyle, hydroxyalkyle, aryle, aryle à substituant(s), alkyl-aryle, alkyl-aryle à substituant(s), hétérocycloalkyle, hétérocycloalkyle à substituant(s), alkyl-hétérocycloalkyle et alkyl-hétérocycloalkyle à substituant(s), les entités symbolisées par -C(=O)OR⁸, -OC(=O)R⁸, -C(=O)NR⁸R⁹, -C(=O)NR⁸OR⁹ -SO_{c}R⁸, -SOcNR⁸R⁹ -NR⁸SO_{c}R⁹ -NR⁸R⁹, -NR⁸C(=O)R⁹, -NR⁸C(=O)(CH₂)_{b}OR⁹, -NR⁸C(=O)(CH₂)_{b}R⁹ et -O(CH₂)_{b}NR⁸R⁹, et les groupes hétérocycloalkyle condensés avec un cycle phényle ;
- R⁸, R⁹, R¹⁰ et R¹¹ représentent des entités identiques ou différentes, qui sont choisies, indépendamment en chaque occurrence, dans l'ensemble formé par un atome d'hydrogène et les groupes alkyle, alkyle à substituant(s), aryle, aryle à substituant(s), alkyl-aryle, alkyl-aryle à substituant(s), hétérocycloalkyle, hétérocycloalkyle à substituant(s), alkyl-hétérocycloalkyle et alkyl-hétérocycloalkyle à substituant(s), ou bien R⁸ et R⁹ représentent des entités qui, conjointement avec l'atome ou les atomes au(x)quel(s) elles sont liées, forment un hétérocycle, en option porteur d'un ou de substituant(s) ;
- les indices a et b ont des valeurs identiques ou différentes, qui sont choisies, indépendamment en chaque occurrence, dans l'ensemble constitué par les nombres 0, 1, 2, 3 et 4 ;
- et l'indice c vaut, en chaque occurrence, 0, 1 ou 2 ;
pour utilisation dans un procédé de traitement et/ou de prévention de l'endométriose chez un individu.

2. Inhibiteur de kinase IKK pour utilisation conforme à la revendication 1, en combinaison avec un répresseur hormonal.

3. Inhibiteur de kinase IKK pour utilisation conforme à la revendication 1 ou 2, pour lequel ledit répresseur hormonal est choisi dans l'ensemble formé par un antagoniste de l'hormone GnRH, un agoniste de GnRH, un inhibiteur de l'aromatase, un modulateur des récepteurs de progestérone et un modulateur des récepteurs d'oestrogènes.

4. Inhibiteur de kinase IKK pour utilisation conforme à l'une des revendications 1 à 3, lequel inhibiteur de kinase IKK est administré seul ou en combinaison avec des médicaments servant au traitement d'une stérilité liée à une endométriose.

5. Inhibiteur de kinase IKK pour utilisation conforme à la revendication 1, dans lequel R⁵ et R⁶ représentent des entités qui, conjointement avec l'atome d'azote auquel elles sont liées, forment un hétérocycle azoté non-aromatique, en option porteur d'un ou de substituant(s).

6. Inhibiteur de kinase IKK pour utilisation conforme à la revendication 1 ou 5, dans lequel l'hétérocycle azoté non-aromatique est choisi dans l'ensemble formé par les groupes suivants : morpholinyle, thiomorpholinyle, pyrrolidinonyle, pyrrolidinyle, pipéridinyle, homopipéridinyle, pipérazinyle, homopipérazinyle, hydantoïnyle, tétrahydropyridinyle, tétrahydropyrimidinyle, oxazolidinyle, thiazolidinyle, indolinyle, isoindolinyle, tétrahydroquinoléinyle et tétrahydroisoquinoléinyle.

7. Inhibiteur de kinase IKK pour utilisation conforme à l'une des revendications 1 et 5 à 6, dans lequel R¹ représente un groupe aryle ou hétéroaryle.

8. Inhibiteur de kinase IKK pour utilisation conforme à l'une des revendications 1 et 5 à 7, dans lequel R¹ représente un groupe choisi dans l'ensemble formé par les suivants : aryle, furyle, benzofuryle, thiényle, benzothiényle, quinoléinyle, pyrrolyle, indolyle, oxazolyle, benzoxazolyle, imidazolyle, benzimidazolyle, thiazolyle, benzothiazolyle, isoxazolyle, pyrazolyle, isothiazolyle, pyridazinyle, pyrimidinyle, pyrazinyle, triazinyle, cinnolinyle, phtalazinyle et quinazolinyle.

9. Inhibiteur de kinase IKK pour utilisation conforme à l'une des revendications 1 et 5 à 8, dans lequel R¹ représente un groupe phényle.

10. Inhibiteur de kinase IKK pour utilisation conforme à l'une des revendications 5 à 9, dans lequel l'hétérocycle azoté est un groupe pipérazinyle.

11. Inhibiteur de kinase IKK pour utilisation conforme à l'une des revendications 5 à 9, dans lequel l'hétérocycle azoté est un groupe pipéridinyle.

12. Inhibiteur de kinase IKK pour utilisation conforme à l'une des revendications 5 à 9, dans lequel l'hétérocycle azoté est un groupe morpholinyle.

13. Inhibiteur de kinase IKK pour utilisation conforme à l'une des revendications 1 à 4, lequel inhibiteur de kinase IKK est un composé de formule (II) : 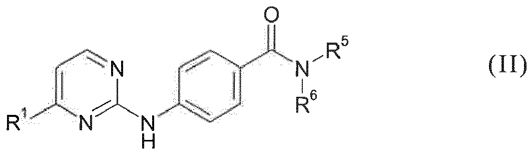 ou l'un de ses sels pharmacologiquement admissibles,
dans laquelle formule
- R¹ représente un groupe aryle ou hétéroaryle, en option porteur de 1 à 4 substituant(s) choisi(s) indépendamment parmi ceux qui sont symbolisés par R⁷ ;
- R⁵ et R⁶ représentent des entités identiques ou différentes, choisies indépendamment dans l'ensemble formé par les entités symbolisées par -R⁸, -(CH₂)ₐC(=O)R⁹, -(CH₂)ₐC(=O)OR⁹, -(CH₂)ₐC(=O)NR⁹R¹⁰, -(CH₂)ₐC(=O)NR⁹(CH₂)_{b}C(=O)R¹⁰, -(CH₂)ₐNR⁹C(=O)R¹⁰, -(CH₂)ₐNR¹¹C(=O)NR⁹R¹⁰, -(CH₂)ₐNR⁹R¹⁰, -(CH₂)ₐOR⁹, -(CH₂)ₐSO_{c}R⁹, et -(CH₂)ₐSO₂NR⁹R¹⁰
ou bien R⁵ et R⁶ représentent des entités qui, conjointement avec l'atome d'azote auquel elles sont liées, forment un hétérocycle, ou un hétérocycle à substituant(s) ;
- R⁷ représente une entité choisie, indépendamment en chaque occurrence, dans l'ensemble formé par les atomes d'halogène, les groupes hydroxyle, cyano, nitro, carboxyle, alkyle, alcoxy, halogéno-alkyle, acyloxy, sulfanyl-alkyle, sulfinyl-alkyle, sulfonyl-alkyle, hydroxyalkyle, aryle, aryle à substituant(s), alkyl-aryle, alkyl-aryle à substituant(s), hétérocycloalkyle, hétérocycloalkyle à substituant(s), alkyl-hétérocycloalkyle et alkyl-hétérocycloalkyle à substituant(s), les entités symbolisées par -C(=O)OR⁸, -OC(=O)R⁸, -C(=O)NR⁸R⁹, -C(=O)NR⁸OR⁹ -SO_{c}R⁸, -SO_{c}NR⁸R⁹ -NR⁸SO_{c}R⁹, -NR⁸R⁹, -NR⁸C(=O)R⁹, -NR⁸C(=O)(CH₂)_{b}OR⁹, -NR⁸C(=O)(CH₂)_{b}R⁹ et -O(CH₂)_{b}NR⁸R⁹, et les groupes hétérocycloalkyle condensés avec un cycle phényle ;
- R⁸, R⁹, R¹⁰ et R¹¹ représentent des entités identiques ou différentes, qui sont choisies, indépendamment en chaque occurrence, dans l'ensemble formé par un atome d'hydrogène et les groupes alkyle, alkyle à substituant(s), aryle, aryle à substituant(s), alkyl-aryle, alkyl-aryle à substituant(s), hétérocycloalkyle, hétérocycloalkyle à substituant(s), alkyl-hétérocycloalkyle et alkyl-hétérocycloalkyle à substituant(s), ou bien R⁸ et R⁹ représentent des entités qui, conjointement avec l'atome ou les atomes au(x)quel(s) elles sont liées, forment un hétérocycle, en option porteur d'un ou de substituant(s) ;
- les indices a et b ont des valeurs identiques ou différentes, qui sont choisies, indépendamment en chaque occurrence, dans l'ensemble constitué par les nombres 0, 1, 2, 3 et 4 ;
- et l'indice c vaut, en chaque occurrence, 0, 1 ou 2.

14. Inhibiteur de kinase IKK pour utilisation conforme à la revendication 13, dans lequel R⁵ et R⁶ représentent des entités qui, conjointement avec l'atome d'azote auquel elles sont liées, forment un hétérocycle azoté non-aromatique, en option porteur d'un ou de substituant(s).

15. Inhibiteur de kinase IKK pour utilisation conforme à la revendication 13 ou 14, dans lequel l'hétérocycle azoté non-aromatique est choisi dans l'ensemble formé par les groupes suivants : morpholinyle, thiomorpholinyle, pyrrolidinonyle, pyrrolidinyle, pipéridinyle, homopipéridinyle, pipérazinyle, homopipérazinyle, hydantoïnyle, tétrahydropyridinyle, tétrahydropyrimidinyle, oxazolidinyle, thiazolidinyle, indolinyle, isoindolinyle, tétrahydroquinoléinyle et tétrahydroisoquinoléinyle.

16. Inhibiteur de kinase IKK pour utilisation conforme à l'une des revendications 13 à 15, dans lequel R¹ représente un groupe aryle ou hétéroaryle.

17. Inhibiteur de kinase IKK pour utilisation conforme à l'une des revendications 13 à 16, dans lequel R¹ représente un groupe choisi dans l'ensemble formé par les suivants : aryle, furyle, benzofuryle, thiényle, benzothiényle, quinoléinyle, pyrrolyle, indolyle, oxazolyle, benzoxazolyle, imidazolyle, benzimidazolyle, thiazolyle, benzothiazolyle, isoxazolyle, pyrazolyle, isothiazolyle, pyridazinyle, pyrimidinyle, pyrazinyle, triazinyle, cinnolinyle, phtalazinyle et quinazolinyle.

18. Inhibiteur de kinase IKK pour utilisation conforme à l'une des revendications 13 à 17, dans lequel R¹ représente un groupe phényle.

19. Inhibiteur de kinase IKK pour utilisation conforme à l'une des revendications 14 à 18, dans lequel l'hétérocycle azoté est un groupe pipérazinyle.

20. Inhibiteur de kinase IKK pour utilisation conforme à l'une des revendications 14 à 18, dans lequel l'hétérocycle azoté est un groupe pipéridinyle.

21. Inhibiteur de kinase IKK pour utilisation conforme à l'une des revendications 14 à 18, dans lequel l'hétérocycle azoté est un groupe morpholinyle.

22. Inhibiteur de kinase IKK pour utilisation conforme à l'une des revendications 1 à 4, lequel inhibiteur de kinase IKK est un composé de formule (III) : 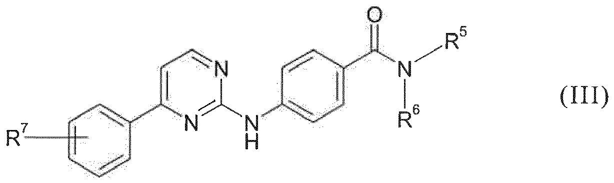 dans laquelle les symboles R⁵, R⁶, R⁷, R⁸, R⁹, R¹⁰ et R¹¹ ont les significations indiquées dans les revendications 13 à 21.

23. Inhibiteur de kinase IKK pour utilisation conforme à l'une des revendications 1 à 4, lequel inhibiteur de kinase IKK est un composé de formule (IV) : 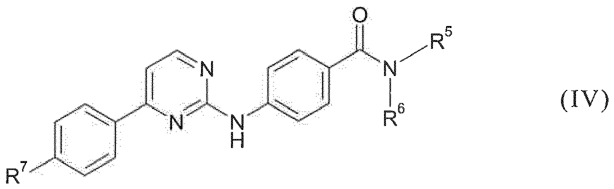 dans laquelle les symboles R⁵, R⁶, R⁷, R⁸, R⁹, R¹⁰ et R¹¹ ont les significations indiquées dans les revendications 13 à 21.

24. Inhibiteur de kinase IKK pour utilisation conforme à l'une des revendications 1 à 4, lequel inhibiteur de kinase IKK est de la 1-(4-{4-[4-(4-chloro-phényl)-pyrimidin-2-yl-amino]-benzoyl}-pipérazin-1-yl)-éthanone.

25. Composition pharmaceutique comprenant un inhibiteur de kinase IKK de formule (I), conforme à la revendication 1, un répresseur hormonal et un excipient pharmacologiquement admissible, pour utilisation dans le traitement et/ou la prévention de l'endométriose chez un individu.

26. Composition pharmaceutique comprenant un inhibiteur de kinase IKK de formule (I), conforme à la revendication 1, et un répresseur hormonal choisi dans l'ensemble constitué par un antagoniste de l'hormone GnRH, un inhibiteur de l'aromatase, et un modulateur des récepteurs de progestérone.

27. Composition pharmaceutique conforme à la revendication 25 ou 26, dans laquelle ledit inhibiteur de kinase IKK est de la 1-(4-{4-[4-(4-chloro-phényl)-pyrimidin-2-yl-amino]-benzoyl}-pipérazin-1-yl)-éthanone.
